# EUROPEAN PATENT APPLICATION

(11) **EP 2 799 034 A1**
(43) Date of publication of application: **05.11.2014**
(21) Application number: 12863442.5
(22) Date of filing: 14.12.2012
(51) Int. Cl.: A61F 2/38

(54) **ARTIFICIAL KNEE JOINT IMPLANT**

(30) Priority: 28.12.2011 JP 2011289762
(71) Applicant: Kyocera Medical Corporation, Osaka-shi, Osaka 532-0003 (JP)
(72) Inventor: MIZUGUCHI,Tomoyuki, Osaka-shi Osaka 532-0003 (JP); HASHIDA,Masahiko, Osaka-shi Osaka 532-0003 (JP)
(74) Representative: Isarpatent
(86) International application number: PCT/JP2012/082562
(87) International publication number: WO 2013/099661

(57) **Abstract**

Provided is an artificial knee joint implant that allows a more natural medial pivot motion to be performed. An artificial knee joint implant 1 includes a femoral component 2 and a tibial plate 4 that are in contact with each other. The femoral component 2 includes a first joint face 11 that is to be disposed adjacent to a medial collateral ligament 105 of a patient, and a second joint face 12 that is to be disposed adjacent to a lateral collateral ligament 106 of the patient. The first joint face 11 and the second joint face 12 respectively have a first medial pivot guide portion 46 and a second medial pivot guide portion 48. A reference face 43 extending orthogonal to the left-right direction is defined between the first joint face 11 and the second joint face 12. An amount ΔD2 of decrease in a distance D2 between the second contact portion 42 on the second medial pivot guide portion 48 and the reference face 43 in accordance with an increase per unit flexion angle is greater than an amount ΔD1 of decrease in a distance D1 between the first contact portion 41 on the first medial pivot guide portion 46 and the reference face 43 in accordance with the increase per unit flexion angle.

## Description

### Technical Field

The present invention relates to an artificial knee joint implant for use in surgery for replacing a knee joint of a patient by an artificial knee joint.

### Background Art

There is a known artificial knee joint including a tibial component fixed to a proximal portion of a tibia and a femoral component fixed to a distal portion of a femur (see Patent Document 1, for example). The femoral component described in Patent Document 1 includes a femoral condyle portion. The femoral condyle portion is configured by a medial condyle portion and a lateral condyle portion that are formed by linking two left and right projecting faces in the antero-posterior direction. The tibial component includes a tibial condyle portion. The tibial condyle portion is configured by a medial condyle portion and a lateral condyle portion. The medial condyle portion and the lateral condyle portion of the tibial component have recess faces. The recess faces support the medial condyle portion and the lateral condyle portion of the femoral component such that these condyle portions can smoothly slide and rotate. In each of the femoral condyle portion and the tibial condyle portion, a distance between the medial condyle portion and the lateral condyle portion decreases toward the posterior side.

### Citation List

### Patent Document

Patent Document 1: JP 2002-28170A ([0007] to [0009])

### Summary of Invention

### Technical Problem

Incidentally, it is known that two motions usually occur in a knee joint of a human body when the knee is extended and flexed. One of the motions is a medial pivot motion, and the other is a motion called rollback.

The medial pivot motion is a motion in which the femur is displaced about the medial condyle portion of the tibia in accordance with an increase in the flexion angle of the knee. The rollback is a motion in which the femur moves to the posterior side while rotating on the tibia during flexion of the knee. Both the medial pivot motion and the rollback are greatly affected by ligaments supporting the knee joint. Of the ligaments supporting the knee joint, an anterior cruciate ligament and a posterior cruciate ligament (hereinafter, also referred to as the ACL and the PCL) for controlling antero-posterior and medial pivot motions of the femur-tibia joint (hereinafter, the F-T joint) are particularly important. In particular, the PCL is known as playing a central role in the medial pivot motion.

In addition to the above, there are a medial collateral ligament and a lateral collateral ligament as ligaments around the knee joint. It is known that, during flexion of the knee, a force of the lateral collateral ligament defining movement of the knee joint is smaller than a force of the medial collateral ligament defining movement of the knee joint. This is one of the reasons why a lateral portion of the knee easily moves due to a medial pivot motion at the time of flexion of the knee joint.

Accordingly, in order to enable a patient with an artificial knee joint to flex his or her knee without a sense of unnaturalness, it is necessary for the artificial knee joint to realize a medial pivot motion in accordance with rollback as the knee is flexed.

In the configuration described in Patent Document 1, as described above, in each of the femoral condyle portion and the tibial condyle portion of the artificial knee joint, a distance between the medial condyle portion and the lateral condyle portion decreases toward the posterior side. Accordingly, as the flexion angle of the knee increases, a distance between a position where the medial condyle portion of the femoral component is in contact with the tibial component and a position where the lateral condyle portion of the femoral component is in contact with the tibial component decreases. As a result, the medial pivot motion of the femoral component easily occurs about the axial line of the tibia relative to the tibial component.

However, although the configuration described in Patent Document 1 allows a medial pivot motion of the tibial component to easily occur relative to the femoral component, this artificial knee joint does not mechanically (actively) facilitate the medial pivot motion of the femur because the medial condyle portion and the lateral condyle portion have the same shape.

The present invention was made in view of these circumstances, and it is an object thereof to provide an artificial knee joint implant that allows a more natural medial pivot motion to be performed.

### Solution to Problem

In order to achieve the above-described object, a first aspect of the present invention is directed to an artificial knee joint implant for use in surgery for replacing a knee joint of a patient by an artificial knee joint, including: a femoral component that is to be attached to a distal portion of a femur of the patient; and a tibial plate that is to be attached to a proximal portion of a tibia of the patient, and guides flexion of the femur relative to the tibia in cooperation with the femoral component; wherein the femoral component includes a first joint face in the shape of a projecting curve that is in contact with the tibial plate at a position adjacent to a medial collateral ligament of the patient, and a second joint face in the shape of a projecting curve that is in contact with the tibial plate at a position adjacent to a lateral collateral ligament of the patient, the first joint face and the second joint face being arranged side by side in a left-right direction, a predetermined reference face extending orthogonal to the left-right direction is defined between the first joint face and the second joint face, the first joint face and the second joint face respectively include a first medial pivot guide portion and a second medial pivot guide portion for guiding a medial pivot motion of the femoral component about an axial line of the tibia, a first contact portion and a second contact portion, which are portions in contact with the tibial plate, of the first joint face and the second joint face are displaced on the first joint face and the second joint face in accordance with a change in a flexion angle of the femur relative to the tibia, and an amount of decrease in a distance between the second contact portion on the second medial pivot guide portion and the reference face in accordance with an increase per unit flexion angle, which is a unit amount of the flexion angle, is greater than an amount of decrease in a distance between the first contact portion on the first medial pivot guide portion and the reference face in accordance with the increase per unit flexion angle.

According to this aspect of the present invention, when the flexion angle increases in a state where the first medial pivot guide portion and the second medial pivot guide portion are in contact with the tibial plate, the amount of shift of the second contact portion toward the reference face is greater than that of the first contact portion toward the reference face. Accordingly, a medial pivot motion of the femur occurs relative to the tibia about the axial line of the tibia. Thus, the artificial knee joint implant can mechanically (actively) cause a medial pivot motion of the femur relative to the tibia in accordance with rollback as the knee is flexed. As a result, the patient can perform a more natural medial pivot motion.

That is to say, the present invention provides an artificial knee joint implant that allows a more natural medial pivot motion to be performed.

A second aspect of the present invention is directed to the artificial knee joint implant according to the first aspect, wherein the tibial plate includes a third joint face that is in contact with the first joint face, and a fourth joint face that is in contact with the second joint face, and the third joint face and the fourth joint face are bilaterally symmetrical to each other.

According to this aspect of the present invention, the third joint face and the fourth joint face are bilaterally symmetrical to each other. Accordingly, the tibial plate can be used either as a tibial plate for a patient's left knee or as a tibial plate for a patient's right knee. Thus, a tibial plate for a patient's left knee and a tibial plate for a patient's right knee do not have to be separately prepared, and the versatility of the tibial plate can be increased. As a result, the production cost of the artificial knee joint implant can be reduced.

A third aspect of the present invention is directed to the artificial knee joint implant according to the first or the second aspect, wherein the amount of decrease in the distance between the second contact portion on the second medial pivot guide portion and the reference face per unit flexion angle, in accordance with an increase in the flexion angle, is the same throughout the second medial pivot guide portion.

According to this aspect of the present invention, when the flexion angle changes in a state where the second medial pivot guide portion is in contact with the tibial plate, the amount of medial pivot motion of the femur with respect to a change by a unit flexion angle can be made more uniform. Accordingly, when the patient's knee is flexed and extended, the medial pivot motion of the femur can be smoothly performed, so that more natural flexion can be performed.

A fourth aspect of the present invention is directed to the artificial knee joint implant according to any one of the first to the third aspects, wherein the amount of decrease in the distance between the first contact portion on the first medial pivot guide portion and the reference face per unit flexion angle, in accordance with an increase in the flexion angle, is the same throughout the first medial pivot guide portion.

According to this aspect of the present invention, when the flexion angle changes in a state where the first medial pivot guide portion is in contact with the tibial plate, the amount of medial pivot motion of the femur with respect to a change by a unit flexion angle can be made more uniform. Accordingly, when the patient's knee is flexed and extended, the medial pivot motion of the femur can be smoothly performed, so that more natural flexion can be performed.

A fifth aspect of the present invention is directed to the artificial knee joint implant according to any one of the first to the fourth aspects, wherein the first medial pivot guide portion and the second medial pivot guide portion come into contact with the tibial plate when the flexion angle is at least a predetermined reference value that is greater than zero.

According to this aspect of the present invention, the flexion angle at which the medial pivot motion of the femur starts can be greater than zero. Accordingly, in a state where the knee is straightened or is nearly straightened, a medial pivot motion of the femur can be prevented from occurring. As a result, a movement state more close to that of a knee joint in a living body can be realized with the artificial knee joint.

A sixth aspect of the present invention is directed to the artificial knee joint implant according to the fifth aspect, wherein, when the flexion angle is less than the reference value, the distance between the first contact portion and the reference face is constant, and the distance between the second contact portion and the reference face is constant, regardless of the flexion angle.

According to this aspect of the present invention, when the flexion angle is zero or a small value, a medial pivot motion in accordance with flexion of the knee is prevented from occurring. Thus, when the flexion angle is zero or a small value, flexion extremely close to that of a knee joint in a living body can be realized with the artificial knee joint.

### Effect of Invention

The present invention provides an artificial knee joint implant that allows a more natural medial pivot motion to be performed.

### Brief Description of Drawings

FIG. 1 is a partial cross-sectional view showing an artificial knee joint implant, a femur of a patient, and a tibia of the patient, viewed from a side of the patient.
FIG. 2(a) is a front view of a femoral component, FIG. 2(b) is a plan view of the femoral component, and FIG. 2(c) is a side view of the femoral component.
FIG. 3 is a rear view of the femoral component.
FIG. 4(a) is a plan view of a tibial plate, FIG. 4(b) is a front view of the tibial plate, and FIG. 4(c) is a side view of the tibial plate.
FIG. 5 shows views of a main portion illustrating flexion of the artificial knee joint implant, where FIG. 5(a) shows a rear view of the femoral component, and FIGS. 5(b) to 5(e) are cross-sectional views each showing a contact state between the femoral component and the tibial plate at a given flexion angle.
FIG. 6 shows views of a main portion illustrating flexion of the artificial knee joint implant, where FIGS. 6(a) to 6(e) each show a plan view and a side view of the femoral component, and a cross-sectional view of the tibial plate at a contact position with the femoral component when viewed from the side, at a given flexion angle.
FIG. 7 shows views of a main portion illustrating flexion of an artificial knee joint implant in a comparative example, where FIG. 7(a) shows a rear view of a femoral component, FIGS. 7(b) to 7(e) are cross-sectional views each showing a contact state between the femoral component and a tibial plate at a given flexion angle, and FIG. 7(f) is a plan view of the tibial plate 204.
FIG. 8 shows views of a main portion illustrating flexion of the artificial knee joint implant in the comparative example, where FIGS. 8(a) to 8(e) each show a plan view and a side view of the femoral component, and a cross-sectional view of the tibial plate at a contact position with the femoral component when viewed from the side, at a given flexion angle.
FIG. 9(a) is a side view of an artificial knee joint implant as a modification of the embodiment of the present invention, and FIG. 9(b) is a plan view of the artificial knee joint implant.

### Description of Embodiment

Hereinafter, an embodiment for carrying out the present invention will be described with reference to the drawings. The present invention is widely applicable as an artificial knee joint implant for use in surgery for replacing a knee joint by an artificial knee joint.

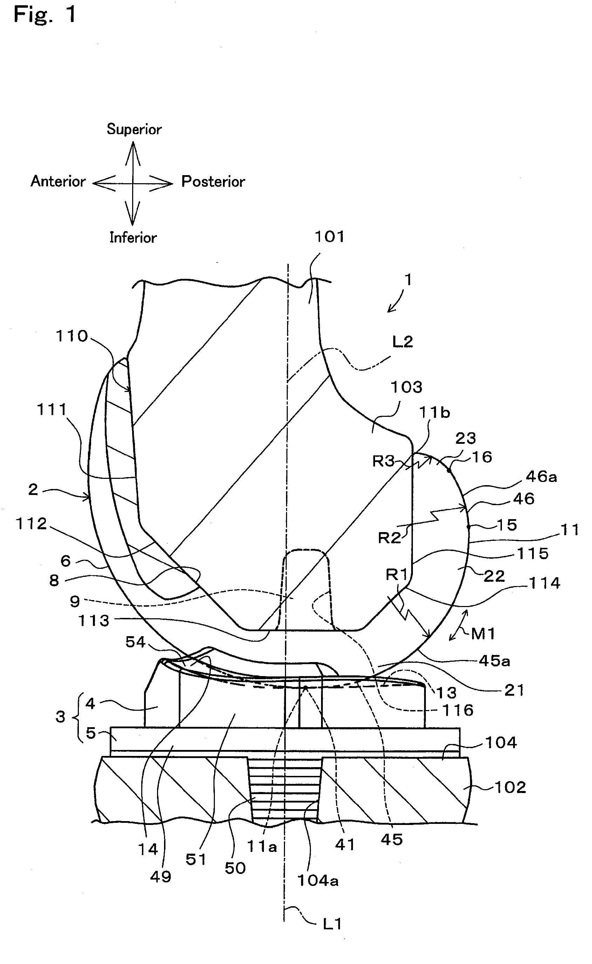

FIG. 1 is a partial cross-sectional view showing an artificial knee joint implant 1, a femur 101 of a patient, and a tibia 102 of the patient, viewed from a side of the patient. In FIG. 1, the femur 101, a femoral component 2 of the artificial knee joint implant 1, and the tibia 102 are shown as cut faces. A tibial component 3 of the artificial knee joint implant 1 is shown as a side face. The artificial knee joint implant 1 is used in surgery for replacing a knee joint of a patient by an artificial knee joint. The artificial knee joint implant 1 is used, for example, to recover normal functions of a patient's knee in which the knee joint has been highly deformed due to osteoarthritis, chronic rheumatoid arthritis, or the like.

In this embodiment, the artificial knee joint implant 1 is used in cruciate retaining (CR) type artificial knee joint replacement surgery for removing an anterior cruciate ligament of a patient while retaining a posterior cruciate ligament, and replacing a knee joint of the patient by an artificial knee joint.

The artificial knee joint implant 1 is configured including the femoral component 2 and the tibial component 3. The tibial component 3 includes a tibial plate 4 and a tibial tray 5.

The femoral component 2 is fixed to a distal portion 103 of the femur 101. The tibial plate 4 is fixed via the tibial tray 5 to a proximal portion 104 of the tibia 102. The femoral component 2 and the tibial component 3 relatively slide over each other as the patient's knee is flexed and extended. In this manner, the femoral component 2 and the tibial component 3, in cooperation with each other, guide flexion of the femur 101 relative to the tibia 102.

In the description below, "medial" and "lateral" respectively refer to the inner side and the outer side with respect to the midline of the body, of the patient's knee on which the artificial knee joint implant 1 is installed. That is to say, if the artificial knee joint implant 1 is disposed on the left leg of the patient, the medial side refers to the right side of the patient, and the lateral side refers to the left side of the patient. Furthermore, "anterior" and "posterior" respectively refer to the front side and the back side of the patient. Also, "superior" and "inferior" respectively refer to the upper side and the lower side of the patient. Unless otherwise specified, the artificial knee joint implant 1 will be described with reference to a state in which the flexion angle θ is zero, that is, a state in which the patient is standing up straight. In this embodiment, a state in which the artificial knee joint implant 1 is attached to the left leg of the patient will be described.

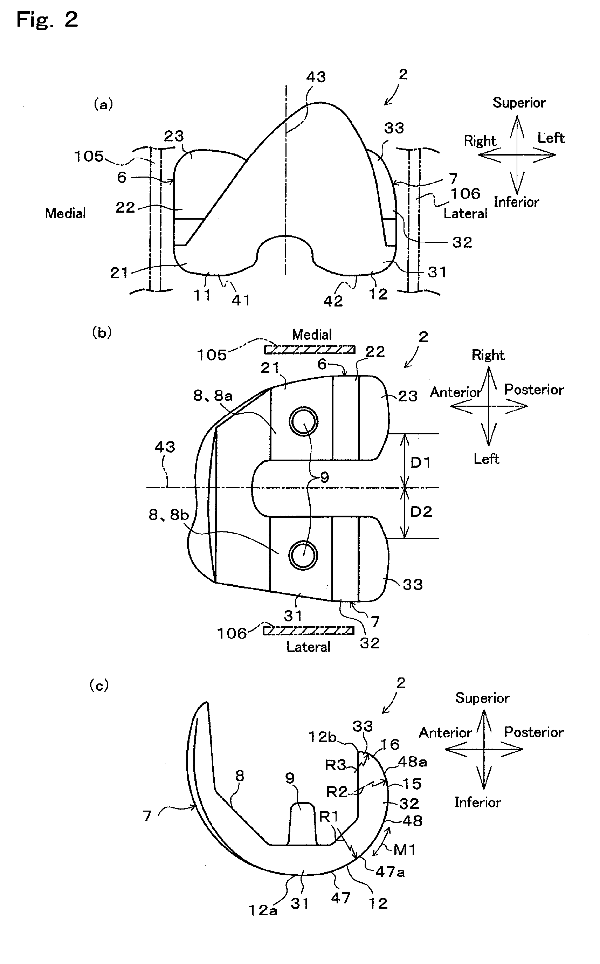

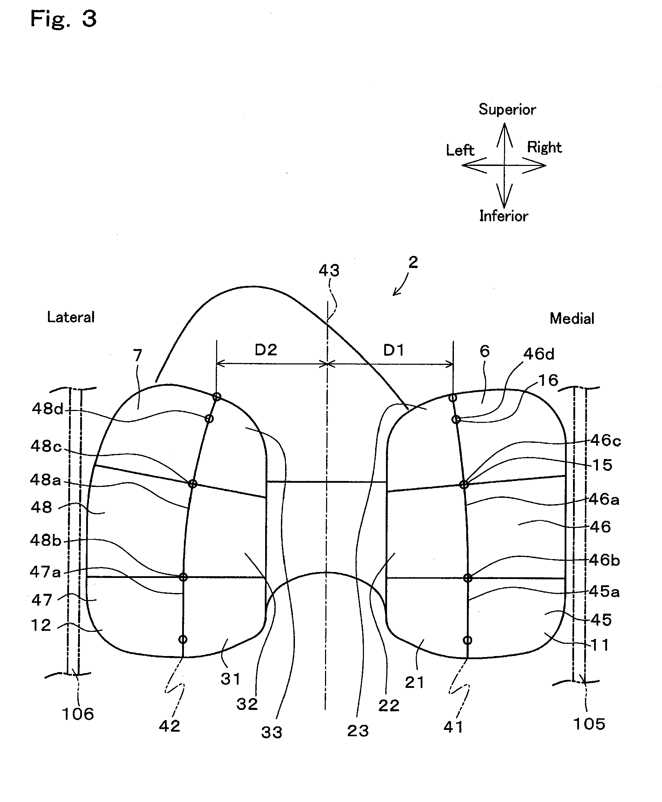

FIG. 2(a) is a front view of the femoral component 2, FIG. 2(b) is a plan view of the femoral component 2, and FIG. 2(c) is a side view of the femoral component 2. FIGS. 2(a) and 2(b) have different corresponding relationships of the orientations in the drawings. FIG. 3 is a rear view of the femoral component 2.

As shown in FIGS. 1, 2(a), 2(b), 2(c), and 3, the femoral component 2 is made of, for example, a metal material having high vital affinity. The femoral component 2 is in the shape of a U when viewed from a side. Furthermore, the femoral component 2 is in the shape of a U when viewed from above.

The femoral component 2 includes a medial condyle 6 and a lateral condyle 7. The medial condyle 6 and the lateral condyle 7 are arranged side by side in the left-right direction. The anterior portion of the medial condyle 6 and the anterior portion of the lateral condyle 7 are connected to each other. The mid-portion and the posterior portion of the medial condyle 6 and the mid-portion and the posterior portion of the lateral condyle 7 are spaced away from each other in the left-right direction, and are arranged approximately parallel to each other in the antero-posterior direction. The anterior portion of the femoral component 2 projects to the superior side when viewed from the front.

The medial condyle 6 and the lateral condyle 7 are provided with, on inner faces thereof facing the distal portion 103 of the femur 101, fixing faces 8. The fixing faces 8 include a fixing face 8a formed on the medial condyle 6 and a fixing face 8b formed on the lateral condyle 7. The fixing faces 8 are provided in order to fix the femoral component 2 to the distal portion 103 of the femur 101. The fixing faces 8 are fixed to a bone resection surface 110 formed on the distal portion 103.

The bone resection surface 110 is a face artificially formed by a surgeon in order to fix the femoral component 2 to the distal portion 103. The bone resection surface 110 is formed, for example, by the surgeon removing part of the distal portion 103 using a tool such as a cutter. The bone resection surface 110 has, for example, a first face 111 oriented to the anterior side, a second face 112 obliquely oriented to the anterior and inferior side, a third face 113 oriented to the inferior side, a fourth face 114 obliquely oriented to the posterior and inferior side, and a fifth face 115 oriented to the posterior side.

The fixing faces 8 are formed in the shape along the first to the fifth faces 111 to 115, and are fixed to the first to the fifth faces 111 to 115 using bone cement, bioactive material coating, or the like. Furthermore, projection portions 9 are formed on the fixing faces 8a and 8b at portions thereof facing the third face 113. The projection portions 9 are inserted into recess portions 116 formed at the third face 113.

The medial condyle 6 and the lateral condyle 7 are provided with, on outer faces thereof facing the side opposite from the distal portion 103 of the femur 101, a first joint face 11 and a second joint face 12. The first joint face 11 and the second joint face 12 are provided as curved faces that slide over the tibial plate 4 as the patient's knee is flexed and extended. The first joint face 11 and the second joint face 12 are arranged side by side in the left-right direction. The first joint face 11 and the second joint face 12 are in the shape of projecting curves projecting toward the tibial plate 4. Each of the first joint face 11 and the second joint face 12 is opposite from a face facing the third face 113, which is a distal end of the femur 101, and is opposite from a face facing the fifth face 115, which is a posterior portion of the distal portion 103, so that the joint faces surround part of the distal portion 103 when viewed from a side.

The first joint face 11 is disposed adjacent to a medial collateral ligament 105 of the patient. The second joint face 12 is disposed adjacent to a lateral collateral ligament 106 of the patient. Usually, of the collateral ligaments 105 and 106, a force of the lateral collateral ligament 106 regulating movement of the artificial knee joint implant 1 is smaller than that of the medial collateral ligament 105.

The first joint face 11 is configured by a distal condyle 21, a posterior condyle 22, and a superior condyle 23 included in the medial condyle 6. The second joint face 12 is configured by a distal condyle 31, a posterior condyle 32, and a superior condyle 33 included in the lateral condyle 7.

The distal condyles 21 and 31 are arranged adjacent to the third face 113, which is a distal face of the femur 101. The posterior condyles 22 and 32 are provided as portions obliquely extending to the posterior and superior side respectively from the distal condyles 21 and 31. Both of the posterior condyles 22 and 32 are mainly arranged adjacent to the fourth face 114 and the fifth face 115. The superior condyles 23 and 33 are provided as portions extending to the superior side respectively from the superior ends of the posterior condyles 22 and 32. The superior condyles 23 and 33 are arranged adjacent to the fifth face 115.

According to the above-described configuration, in this embodiment, the first joint face 11 and the second joint face 12 are respectively configured by outer faces of part of posterior portions of the distal condyles 21 and 31, outer faces of the posterior condyles 22 and 32, and outer faces of the superior condyles 23 and 33. The femoral component 2 is displaced in a displacement direction M1 relative to the tibial plate 4, when viewed from a side. The displacement direction M1 is a direction along the first joint face 11 and the second joint face 12 when viewed from a side.

Ends 11a and 12a, on one of the sides in the displacement direction M1, of the first joint face 11 and the second joint face 12 are positioned at the inferior end of the femoral component 2 and respectively adjacent to the projection portions 9 in the superior-inferior direction. Further, ends 11b and 12b, on the other side in the displacement direction M1, of the first joint face 11 and the second joint face 12 are positioned respectively adjacent to the superior ends of the posterior portions of the fixing faces 8.

Each of the first joint face 11 and the second joint face 12 is configured by arc-shaped faces having a plurality of radii of curvature. In this embodiment, the first joint face 11 and the second joint face 12 have radii of curvature R1, R2, and R3 when viewed from a side.

On the first joint face 11 and the second joint face 12, part of posterior portions of the distal condyles 21 and 31 and part of inferior portions of the posterior condyles 22 and 32 have the radius of curvature R1. Posterior ends 15 of the portions having the radius of curvature R1 on the first joint face 11 and the second joint face 12 are positioned posterior to the fixing faces 8. The posterior ends 15 are positioned at the posterior end of the femoral component 2.

On the first joint face 11 and the second joint face 12, portions extending from the posterior ends 15 to the superior side have the radius of curvature R2 that is smaller than the radius of curvature R1. The portions having the radius of curvature R2 extend to the superior ends of the posterior condyles 22 and 32. That is to say, superior ends 16 of the portions having the radius of curvature R2 are positioned at the superior ends of the posterior condyles 22 and 32. Portions extending from the superior ends 16 to the superior side have the radius of curvature R3 that is smaller than the radius of curvature R2. The portions having the radius of curvature R3 are configured by the superior condyles 23 and 33, and extend to the ends 11b and 12b. According to the above-described configuration, the radius of curvature R1 > R2 > R3.

Furthermore, the first joint face 11 is curved such that a mid-portion thereof in the left-right direction projects toward the outer side of the femoral component 2. In a similar manner, the second joint face 12 is curved such that a mid-portion thereof in the left-right direction projects toward the outer side of the femoral component 2. With this configuration, the mid-portions in the left-right direction of the first joint face 11 and the second joint face 12 are in contact with the tibial plate 4.

The portion of the first joint face 11 in contact with the tibial plate 4 is a first contact portion 41. The portion of the second joint face 12 in contact with the tibial plate 4 is a second contact portion 42. That is to say, the first contact portion 41 is positioned at the top of the first joint face 11, which is the mid-portion in the left-right direction. In a similar manner, the second contact portion 42 is positioned at the top of the second joint face 12, which is the mid-portion in the left-right direction. The first contact portion 41 and the second contact portion 42 are displaced respectively on the first joint face 11 and the second joint face 12 in accordance with a change in the flexion angle θ of the femur 103 relative to the tibia 102.

A reference face 43 extending orthogonal to the left-right direction is defined between the first joint face 11 and the second joint face 12. The reference face 43 is a virtual plane defined between the first contact portion 41 and the second contact portion 42. The reference face 43 is regarded, for example, as a virtual plane that is disposed approximately at the center in the left-right direction between the lateral condyle 7 and the medial condyle 6, and whose position does not change regardless of flexion of the artificial knee joint implant 1. That is to say, the reference face 43 is a plane whose position with respect to the tibial plate 4 does not change regardless of the flexion angle θ.

In this embodiment, the flexion angle θ as an angle of an axial line L2 of the femur 101 with respect to an axial line L1 of the tibia 102 when viewed from a side (hereinafter, alternatively referred to simply as a "flexion angle θ") is defined. The manner in which a distance between the first contact portion 41 and the reference face 43 changes in accordance with a change in the flexion angle θ is different from the manner in which the distance between the second contact portion 42 and the reference face 43 changes at that time. FIG. 1 shows a state in which the flexion angle θ is zero.

The first joint face 11 has a first straight motion guide portion 45 and a first medial pivot guide portion 46. The second joint face 12 has a second straight motion guide portion 47 and a second medial pivot guide portion 48.

The first straight motion guide portion 45 and the second straight motion guide portion 47 are provided in order to move the femoral component 2 in a direction parallel to the reference face 43 during flexion of the artificial knee joint implant 1. When the flexion angle θ is less than a predetermined reference value θ1, the first contact portion 41 and the second contact portion 42 are positioned at the first straight motion guide portion 45 and the second straight motion guide portion 47. In this embodiment, the reference value θ1 is set to, for example, 45 degrees. That is to say, the first contact portion 41 and the second contact portion 42 are positioned at the first straight motion guide portion 45 and the second straight motion guide portion 47 when the flexion angle θ is zero or more and less than 45 degrees.

The first straight motion guide portion 45 includes a portion formed on the distal condyle 21 and a portion formed on part of the posterior condyle 22, on the first joint face 11. A top portion 45a of the first straight motion guide portion 45 includes the portion having the radius of curvature R1 when viewed from a side. The top portion 45a of the first straight motion guide portion 45 extends approximately parallel to the reference face 43. When the first contact portion 41 is positioned at the top portion 45a due to contact of the top portion 45a of the first straight motion guide portion 45 with the tibial plate 4, a distance D1 between the first contact portion 41 and the reference face 43 is constant regardless of the flexion angle θ.

The second straight motion guide portion 47 is bilaterally symmetrical to the first straight motion guide portion 45. That is to say, the second straight motion guide portion 47 includes a portion formed on the distal condyle 31 and a portion formed on part of the posterior condyle 32, on the second joint face 12. A top portion 47 of the second straight motion guide portion 47 includes the portion having the radius of curvature R1 when viewed from a side. The top portion 47a of the second straight motion guide portion 47 extends approximately parallel to the reference face 43. When the second contact portion 42 is positioned at the top portion 47a due to contact of the top portion 47a of the second straight motion guide portion 47 with the tibial plate 4, a distance D2 between the second contact portion 42 and the reference face 43 is constant regardless of the flexion angle θ. It is assumed that distance D1 = distance D2. The first medial pivot guide portion 46 and the second medial pivot guide portion 48 are arranged adjacent to the first straight motion guide portion 45 and the second straight motion guide portion 47 having the above-described configuration.

The first medial pivot guide portion 46 and the second medial pivot guide portion 48 are provided in order to cause a medial pivot motion of the femoral component 2 relative to the axial line L1 of the tibia 102 during flexion of the artificial knee joint implant 1. The first contact portion 41 and the second contact portion 42 are positioned at the first medial pivot guide portion 46 and the second medial pivot guide portion 48 when the flexion angle θ is at least the predetermined reference value θ1. The reference value θ1 is set to, for example, approximately 45 degrees. Note that the upper limit value of the flexion angle θ is approximately a hundred and several tens of degrees although it varies depending on the shapes of the distal portion 103 of the femur 101 and the proximal portion 104 of the tibia 102 of the patient.

The first medial pivot guide portion 46 is formed on part of the posterior condyle 22 and the superior condyle 23 on the first joint face 11. The first contact portion 41 is positioned at a top portion 46a of the first medial pivot guide portion 46. The top portion 46a of the first medial pivot guide portion 46 includes the portions having the radii of curvature R1, R2, and R3 when viewed from a side. The distance between the top portion 46a of the first medial pivot guide portion 46 and the reference face 43 varies depending on the position. More specifically, the top portion 46a shifts toward the reference face 43 as being away from the first straight motion guide portion 45 in the displacement direction M1. In other words, the distance between the top portion 46a and the reference face 43 decreases as being away from the first straight motion guide portion 45 in the displacement direction M1. Accordingly, when the first contact face 41 is positioned at the top portion 46a of the first medial pivot guide portion 46, the distance D1 between the first contact portion 41 and the reference face 43 decreases in accordance with an increase in the flexion angle θ. In this embodiment, an amount ΔD1 of decrease in the distance D1 between the first contact portion 41 and the reference face 43 per unit flexion angle, in accordance with an increase in the flexion angle θ when the first contact portion 41 is positioned at the top portion 46a, is the same throughout the top portion 46a.

An end 46b in the displacement direction M1 of the top portion 46a continues to the top portion 45a of the first straight motion guide portion 45. The first contact portion 41 is positioned at the end 46b, for example, when the flexion angle θ is 45 degrees. Furthermore, in a first mid-portion 46c in the displacement direction M1 of the top portion 46a, the radius of curvature of the top portion 46a when viewed from a side changes from R1 to R2. The first contact portion 41 is positioned at the first mid-portion 46c, for example, when the flexion angle θ is 60 degrees. Furthermore, in a second mid-portion 46d in the displacement direction M1 of the top portion 46a, the radius of curvature of the top portion 46a when viewed from a side changes from R2 to R3. The first contact portion 41 is positioned at the second mid-portion 46d, for example, when the flexion angle θ is 90 degrees. The second medial pivot guide portion 48 is disposed adjacent in the left-right direction to the first medial pivot guide portion 46 having the above-described configuration.

The second medial pivot guide portion 48 is formed on part of the posterior condyle 32 and the superior condyle 33 on the second joint face 12. Atop portion 48a of the second medial pivot guide portion 48 forms the second contact portion 42. The top portion 48a of the second medial pivot guide portion 48 includes the portions having the radii of curvature R1, R2, and R3 when viewed from a side. The distance between the top portion 48a of the second medial pivot guide portion 48 and the reference face 43 varies depending on the position. More specifically, the top portion 48a shifts toward the reference face 43 as being away from the second straight motion guide portion 47 in the displacement direction M1. In other words, the distance between the top portion 48a and the reference face 43 decreases as being away from the second straight motion guide portion 47 in the displacement direction M1. Accordingly, when the second contact portion 42 is positioned at the top portion 48a of the second medial pivot guide portion 48, the distance D2 between the second contact portion 42 and the reference face 43 decreases in accordance with an increase in the flexion angle θ. In this embodiment, an amount ΔD2 of decrease in the distance D2 between the second contact portion 42 and the reference face 43 per unit flexion angle, in accordance with an increase in the flexion angle θ when the second contact face 42 is positioned at the top portion 48a, is the same throughout the top portion 48a.

An end 48b in the displacement direction M1 of the top portion 48a continues to the top portion 47a of the second straight motion guide portion 47. The end 48b of the top portion 48a and the end 46b of the top portion 46a are arranged side by side in the left-right direction. Furthermore, in a first mid-portion 48c in the displacement direction M1 of the top portion 48a, the radius of curvature of the top portion 48a when viewed from a side changes from R1 to R2. The first mid-portion 48c and the first mid-portion 46c are arranged side by side in the left-right direction. The second contact portion 42 is positioned at the first mid-portion 48c, for example, when the flexion angle θ is 60 degrees. Furthermore, in a second mid-portion 48d in the displacement direction M1 of the top portion 48a, the radius of curvature of the top portion 48a when viewed from a side changes from R2 to R3. The second mid-portion 48d and the second mid-portion 46d are arranged side by side in the left-right direction. The second contact portion 42 is positioned at the second mid-portion 48d, for example, when the flexion angle θ is 90 degrees.

In this embodiment, the degree of curve in the top portion 48a of the second medial pivot guide portion 48 in a rear view is greater than that of the top portion 46a of the first medial pivot guide portion 46. As described above, the amount of decrease in the distance D1 between the first contact portion 41 on the first medial pivot guide portion 46 and the reference face 43 in accordance with an increase per unit flexion angle is ΔD1. Furthermore, the amount of decrease in the distance D2 between the second contact portion 42 on the second medial pivot guide portion 48 and the reference face 43 in accordance with an increase per unit flexion angle is ΔD2. In this embodiment, the amount ΔD2 of decrease is greater than the amount ΔD1 of decrease (ΔD2 > ΔD1). The femoral component 2 having the above-described configuration is slidably supported by the tibial component 3.

The tibial tray 5 of the tibial component 3 includes a tray main body 49 that supports the tibial plate 4, and a stud portion 50 that projects from the bottom face of the tray main body 49. The stud portion 50 is fitted into a hole portion 104a formed in the proximal portion 104 of the tibia 102, and is fixed to the proximal portion 104 of the tibia 102 using bone cement, bioactive material coating, or the like. The tray main body 49 is in the shape of a flat plate, and is disposed on the end face of the proximal portion 104. The tray main body 49 has a surface facing the femoral component 2. The tibial plate 4 is fixed to this surface of the tray main body 49.

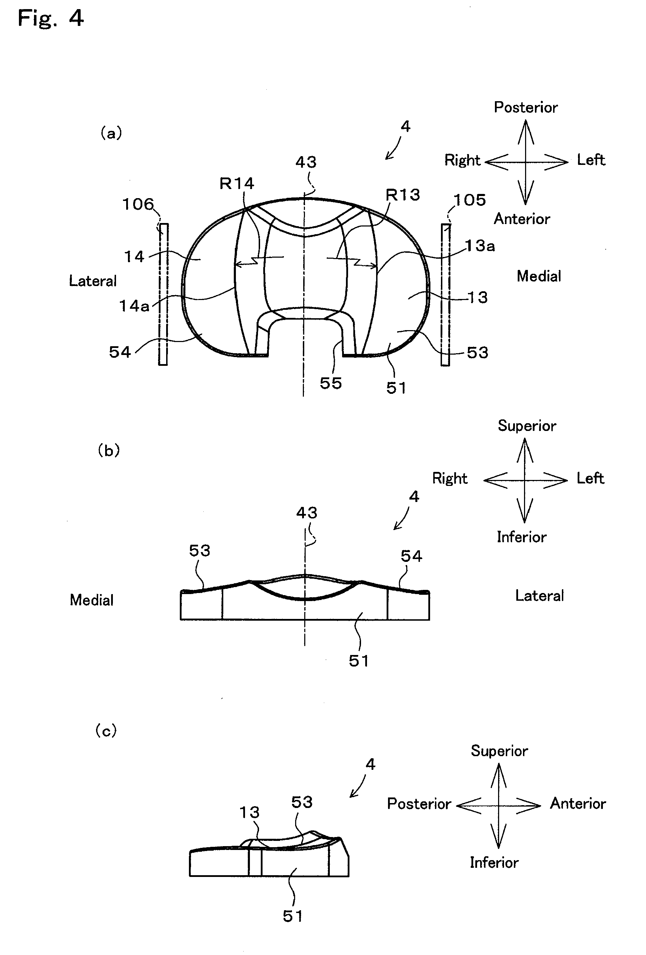

FIG. 4(a) is a plan view of the tibial plate 4, FIG. 4(b) is a front view of the tibial plate 4, and FIG. 4(c) is a side view of the tibial plate 4. As shown in FIGS. 1, 3, 4(a), 4(b), and 4(c), the tibial plate 4 is made of a synthetic resin or the like. The tibial plate 4 has a flat plate 51. The plate 51 is in the shape of a disk that is elongated in the left-right direction. The plate 51 is provided with a medial recess 53 and a lateral recess 54.

The medial recess 53 and the lateral recess 54 are provided as depressions that are in slidable contact respectively with the medial condyle 6 and the lateral condyle 7 of the femoral component 2. A third joint face 13 is formed on a face of the medial recess 53 facing the medial condyle 6 of the femoral component 2. In a similar manner, a fourth joint face 14 is formed on a face of the lateral recess 54 facing the lateral condyle 7 of the femoral component 2.

The third joint face 13 is provided so as to be in contact with the first joint face 11 of the medial condyle 6. The fourth joint face 14 is provided so as to be in contact with the second joint face 12 of the lateral condyle 7. The contact state between the third joint face 13 and the first joint face 11 includes one or both of rolling contact and sliding contact. In a similar manner, the contact state between the fourth joint face 14 and the second joint face 12 includes one or both of rolling contact and sliding contact.

A bottom portion 13a of the third joint face 13 is curved when viewed from above. More specifically, the bottom portion 13a of the third joint face 13 has a predetermined radius of curvature R13 when viewed from above. The bottom portion 13a is in contact with the first contact portion 41 on the first joint face 11. The fourth joint face 14 is formed adjacent in the left-right direction to the third joint face 13.

The fourth joint face 14 is bilaterally symmetrical to the third joint face 13. In other words, the fourth joint face 14 is symmetrical to the third joint face 13 with respect to the reference face 43. Specifically, a bottom portion 14a of the fourth joint face 14 is curved when viewed from above. More specifically, the bottom portion 14a of the fourth joint face 14 has a predetermined radius of curvature R14 when viewed from above. The bottom portion 14a is in contact with the second contact portion 42 on the second joint face 12. A cut-out portion 55 is formed in the posterior portion of the plate 51. The cut-out portion 55 is formed at the center in the left-right direction of the posterior end of the plate 51.

### Description of Flexion of the Artificial Knee Joint

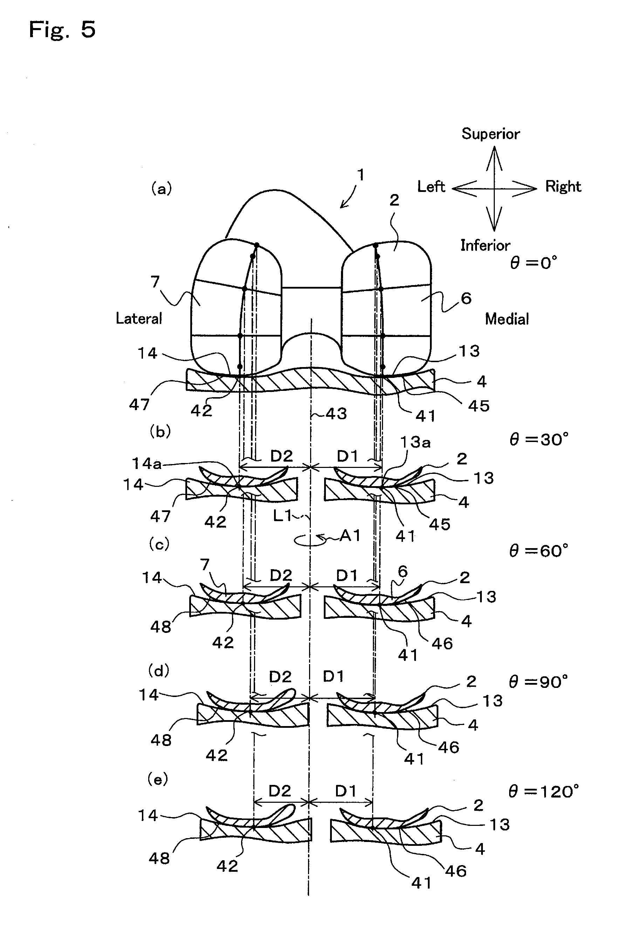

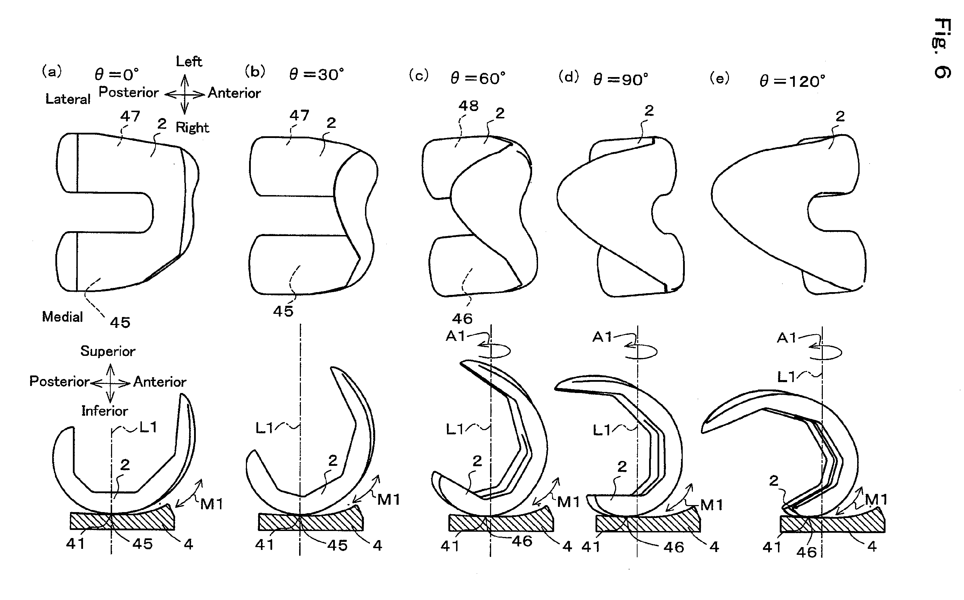

FIG. 5 shows views of a main portion illustrating flexion of the artificial knee joint implant 1, where FIG. 5(a) shows a rear view of the femoral component 2, and FIGS. 5(b) to 5(e) are cross-sectional views each showing a contact state between the femoral component 2 and the tibial plate 4 at a given flexion angle θ. Furthermore, FIG. 6 shows views of a main portion illustrating flexion of the artificial knee joint implant 1, where FIGS. 6(a) to 6(e) each show a plan view and a side view of the femoral component 2, and a cross-sectional view of the tibial plate 4 at a contact position with the femoral component 2 when viewed from the side, at a given flexion angle θ.

As shown in FIGS. 5(a) and 6(a), when the flexion angle θ is zero, the first straight motion guide portion 45 of the femoral component 2 is in contact with the third joint face 13, and, thus, the first contact portion 41 is positioned at the first straight motion guide portion 45. Furthermore, the second straight motion guide portion 47 of the femoral component 2 is in contact with the fourth joint face 14, and, thus, the second contact portion 42 is positioned at the second straight motion guide portion 47.

When the femoral component 2 is displaced to one side in the displacement direction M1 relative to the tibial plate 4 from this state, the flexion angle θ becomes greater than zero. For example, when the flexion angle θ is 30 degrees as shown in FIGS. 5(b) and 6(b), the first straight motion guide portion 45 and the second straight motion guide portion 47 of the femoral component 2 are in contact respectively with the third joint face 13 and the fourth joint face 14. Accordingly, the first contact portion 41 and the second contact portion 42 are positioned respectively at the first straight motion guide portion 45 and the second straight motion guide portion 47. When the first straight motion guide portion 45 and the second straight motion guide portion 47 are in contact respectively with the third joint face 13 and the fourth joint face 14 in this manner, the distance D1 between the first contact portion 41 and the reference face 43 and the distance D2 between the second contact portion 42 and the reference face 43 are the same regardless of the flexion angle θ.

When the flexion angle θ increases to reach at least the predetermined reference value θ1 (45 degrees in this embodiment), the first medial pivot guide portion 46 and the second medial pivot guide portion 48 of the femoral component 2 come into contact respectively with the third joint face 13 and the fourth joint face 14 as shown in FIGS. 5(c) and 6(c). Accordingly, the first contact portion 41 and the second contact portion 42 are positioned respectively at the first medial pivot guide portion 46 and the second medial pivot guide portion 48. In this case, the amount ΔD2 of decrease in the distance D2 in accordance with an increase in the flexion angle θ by unit flexion angle, that is, by one degree is greater than the amount ΔD1 of decrease in the distance D1. As a result, the distance D2 is shorter than the distance D1. Accordingly, in accordance with an increase in the flexion angle θ, a medial pivot motion of the femoral component 2 occurs such that the lateral condyle 7 of the femoral component 2 moves toward the reference face 43. That is to say, a medial pivot motion of the femoral component 2 occurs about the axial line L1 of the tibia 102 in the direction of arrow A1.

When the flexion angle θ further increases from the state shown in FIGS. 5(c) and 6(c), the medial pivot motion of the femoral component 2 progresses about the axial line L1 as shown in FIGS. 5(d) and 6(d). At that time, the medial pivot motion of the femoral component 2 progresses in the direction of arrow A1 in a uniform amount, for each increase in the flexion angle θ by a unit flexion angle.

As a result, when the flexion angle θ is 120 degrees that is close to the upper limit value as shown in FIGS. 5(e) and 6(e), the amount of medial pivot motion of the femoral component 2 about the axial line L1 becomes sufficient. In this manner, the motion in which the femoral component 2 moves to the posterior side while rotating over the tibial plate 4 (rolling contact and sliding contact) in accordance with flexion of the knee, that is, rollback occurs.

Note that, when the flexion angle θ decreases from the state as shown in FIGS. 5(e) and 6(e) where the flexion angle θ is 120 degrees, that is, at the time of extension of the knee, the artificial knee joint implant 1 moves in a manner opposite to the above.

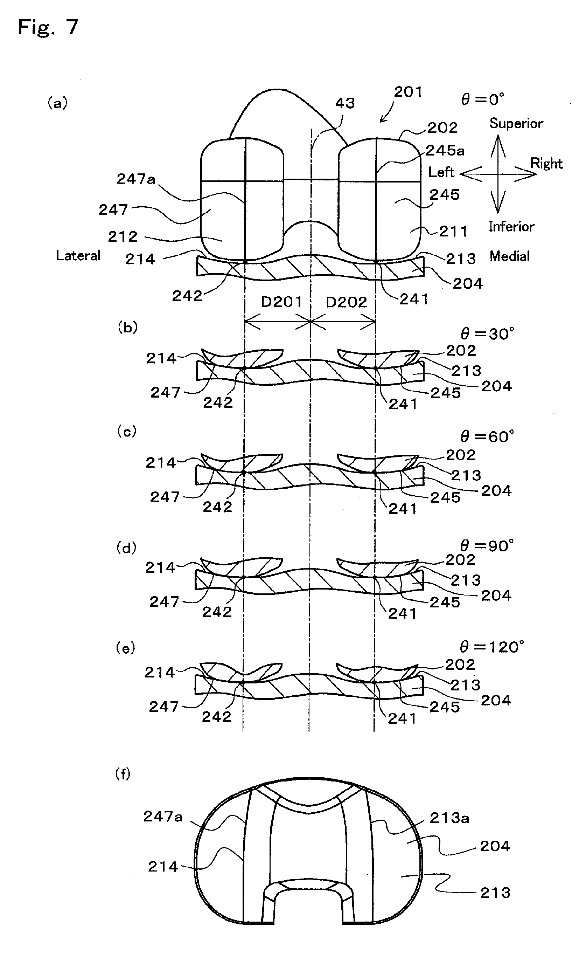

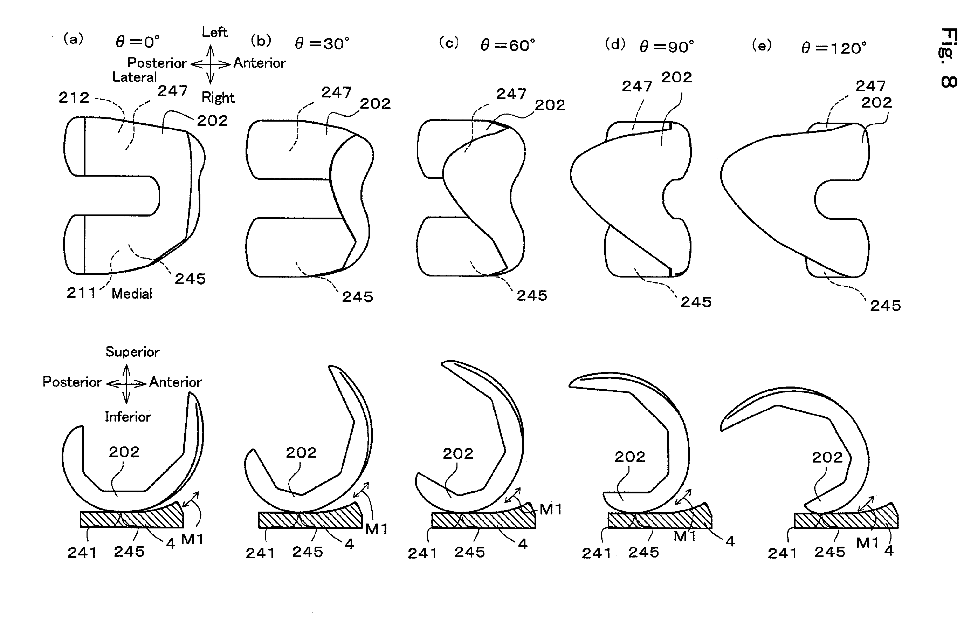

Next, a movement of an artificial knee joint implant 201, which is a comparative example of the artificial knee joint implant 1, will be described with reference to FIGS. 7 and 8. FIG. 7 shows views of a main portion illustrating flexion of the artificial knee joint implant 201, where FIG. 7(a) shows a rear view of a femoral component 202, FIGS. 7(b) to 7(e) are cross-sectional views each showing a contact state between the femoral component 202 and a tibial plate 204 at a given flexion angle θ, and FIG. 7(f) is a plan view of the tibial plate 204. Furthermore, FIG. 8 shows views of a main portion illustrating flexion of the artificial knee joint implant 201, where FIGS. 8(a) to 8(e) each show a plan view and a side view of the femoral component 202, and a cross-sectional view of the tibial plate 204 at a contact position with the femoral component 202 when viewed from the side, at a given flexion angle θ.

The artificial knee joint implant 201 has the femoral component 202 and the tibial plate 204. A first joint face 211 of the femoral component 202 is configured by a first straight motion guide portion 245. Furthermore, a second joint face 212 is configured by a second straight motion guide portion 247. That is to say, the femoral component 202 has the same configuration as the femoral component 2, except that no medial pivot guide portion is provided.

Atop portion 245a of the first straight motion guide portion 245 extends parallel to the reference face 43. Furthermore, the second straight motion guide portion 247 is bilaterally symmetrical to the first straight motion guide portion 245, and has a top portion 247a that extends parallel to the reference face 43. As shown in FIG. 7(f), the tibial plate 204 that supports the femoral component 202 has a third joint face 213 and a fourth joint face 214. The tibial plate 4 and the tibial plate 204 have the same configuration, except that the third joint faces and the fourth joint faces have different shapes.

When viewed from above, a bottom portion 213a of the third joint face 213 has an anterior half portion in the shape of an arc, and a posterior half portion extending in a straight line parallel to the reference face 43. The fourth joint face 214 is bilaterally symmetrical to the third joint face 213. That is to say, when viewed from above, a bottom portion 214a of the fourth joint face 214 has an anterior half portion in the shape of an arc, and a posterior half portion extending in a straight line parallel to the reference face 43.

As shown in FIGS. 7(a) and 8(a), when the flexion angle θ is zero, the first straight motion guide portion 245 of the femoral component 202 is in contact with the third joint face 213, and, thus, a first contact portion 241 is positioned at the first straight motion guide portion 245. Furthermore, the second straight motion guide portion 247 of the femoral component 202 is in contact with the fourth joint face 214, and, thus, a second contact portion 242 is positioned at the second straight motion guide portion 247.

When the femoral component 202 is displaced to one side in the displacement direction M1 relative to the tibial plate 204 from this state, the flexion angle θ becomes greater than zero as shown in FIGS. 7(b) and 8(b). However, regardless of the flexion angle θ, the first straight motion guide portion 245 and the second straight motion guide portion 247 of the femoral component 202 are in contact respectively with the third joint face 213 and the fourth joint face 214. Accordingly, a distance D201 between the first contact portion 241 and the reference face 43 and a distance D202 between the second contact portion 242 and the reference face 43 are constant regardless of the flexion angle θ.

That is to say, the distances D201 and D202 are the same in all of the cases where the flexion angle θ is 30 degrees shown in FIGS. 7(b) and 8(b), where the flexion angle θ is 60 degrees shown in FIGS. 7(c) and 8(c), where the flexion angle θ is 90 degrees shown in FIGS. 7(d) and 8(d), and where the flexion angle θ is 120 degrees shown in FIGS. 7(e) and 8(e). Accordingly, no medial pivot motion of the femoral component 202 occurs relative to the tibial plate 4.

As described above, according to the artificial knee joint implant 1, the first medial pivot guide portion 46 and the second medial pivot guide portion 48 may be in contact with the third joint face 13 and the fourth joint face 14 of the tibial plate 4. In this case, the amount of shift of the second contact portion 42 toward the reference face 43 in accordance with an increase in the flexion angle θ is greater than that of the first contact portion 41 toward the reference face 43. Accordingly, a medial pivot motion of the femur 101 occurs relative to the tibia 102 about the axial line L1 of the tibia 102. Thus, the artificial knee joint implant 1 can mechanically (actively) cause a medial pivot motion of the femur 101 relative to the tibia 102 in accordance with rollback as the patient's knee is flexed. As a result, a more natural medial pivot motion can be performed.

Accordingly, the artificial knee joint implant 1 allows a more natural medial pivot motion to be performed.

Furthermore, according to the artificial knee joint implant 1, the third joint face 13 and the fourth joint face 14 of the tibial plate 4 are bilaterally symmetrical to each other. Accordingly, the tibial plate 4 can be used either as a tibial plate for a patient's left knee or as a tibial plate for a patient's right knee. Thus, a tibial plate for a patient's left knee and a tibial plate for a patient's right knee do not have to be separately prepared, and the versatility of the tibial plate 4 can be increased. As a result, the production cost of the artificial knee joint implant 1 can be reduced.

Furthermore, according to the artificial knee joint implant 1, the amount ΔD2 of decrease in the distance D2 between the second contact portion 42 on the second medial pivot guide portion 48 and the reference face 43 per unit flexion angle, in accordance with an increase in the flexion angle θ, is the same throughout the second medial pivot guide portion 48 along the displacement direction M1. With this configuration, when the flexion angle θ changes in a state where the second medial pivot guide portion 48 is in contact with the tibial plate 4, the amount of medial pivot motion of the femur 101 with respect to a change by a unit flexion angle can be made more uniform. Accordingly, when the patient's knee is flexed and extended, the medial pivot motion of the femur 101 can be smoothly performed, so that more natural flexion can be performed.

Furthermore, according to the artificial knee joint implant 1, the amount ΔD1 of decrease in the distance D1 between the first contact portion 41 on the first medial pivot guide portion 46 and the reference face 43 per unit flexion angle, in accordance with an increase in the flexion angle θ, is the same throughout the first medial pivot guide portion 46. With this configuration, when the flexion angle θ changes in a state where the first medial pivot guide portion 46 is in contact with the tibial plate 4, the amount of medial pivot motion of the femur 101 with respect to a change by a unit flexion angle can be made more uniform. Accordingly, when the patient's knee is flexed and extended, the medial pivot motion of the femur 101 can be more smoothly performed, so that more natural flexion can be performed.

Furthermore, according to the artificial knee joint implant 1, when the flexion angle θ is at least the predetermined reference value θ1 that is greater than zero, the first medial pivot guide portion 46 and the second medial pivot guide portion 48 are in contact with the tibial plate 4. Accordingly, the flexion angle θ at which the medial pivot motion of the femur 101 starts can be greater than zero. Accordingly, in a state where the knee is straightened or is nearly straightened, a medial pivot motion of the femur 101 can be prevented from occurring. As a result, a movement state more close to that of a knee joint in a living body can be realized with the artificial knee joint implant 1.

Furthermore, according to the artificial knee joint implant 1, when the flexion angle θ is less than the predetermined reference value θ1, the distance D1 between the first contact portion 41 and the reference face 43 is constant, and the distance D2 between the second contact portion 42 and the reference face 43 is constant, regardless of the flexion angle θ. Accordingly, when the flexion angle θ is zero or a small value, a medial pivot motion in accordance with flexion of the knee is prevented from occurring. In this manner, when the flexion angle θ is zero or a small value, flexion extremely close to that of a knee joint in a living body can be realized with the artificial knee joint implant 1.

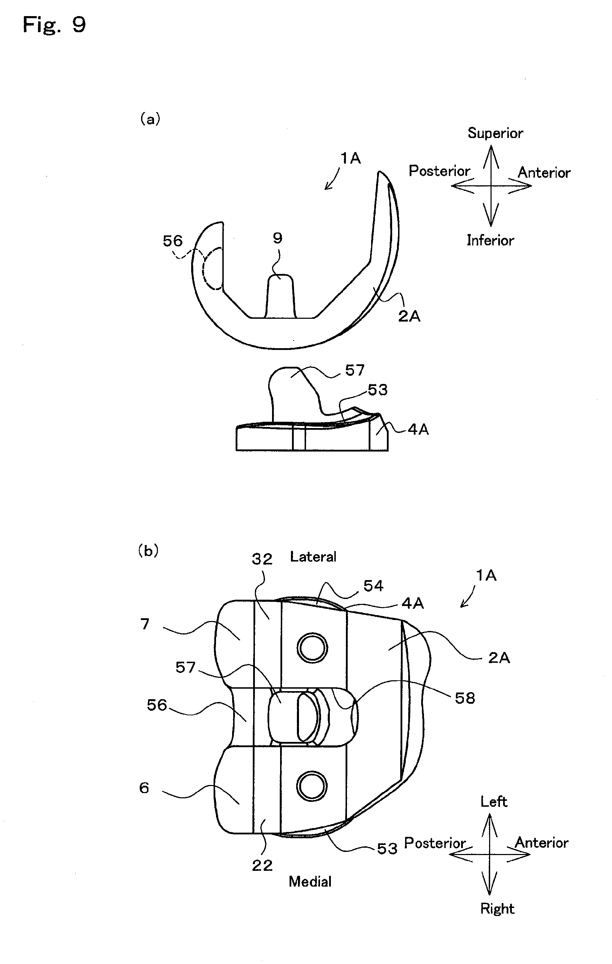

In the description above, an embodiment of the present invention was described, but the present invention is not limited thereto, and various modifications may be made within the scope recited in the claims. For example, the following modifications are possible.
(1) Although the foregoing embodiment was described using an example in which the artificial knee joint implant is a CR-type artificial knee implant, there is no limitation to this. For example, the present invention may be applied to a posterior-stabilized (PS) type artificial knee joint implant 1A shown in FIGS. 9(a) and 9(b). The PS-type artificial knee joint implant 1A is used in artificial knee joint replacement surgery in which both an anterior cruciate ligament and a posterior cruciate ligament of a patient are removed.
   FIG. 9(a) is a side view of the artificial knee joint implant 1A as a modification of the embodiment of the present invention, and FIG. 9(b) is a plan view of the artificial knee joint implant 1A. In the description below, a description will be given mainly of differences from the foregoing embodiment, and the same constituent elements as those in the foregoing embodiment are denoted by the same reference numerals and a description thereof has been omitted.
   The artificial knee joint implant 1A includes a femoral component 2A and a tibial plate 4A. The femoral component 2A is different from the femoral component 2 in that a cam portion 56 is provided. Furthermore, the tibial plate 4A is different from the tibial plate 4 in that a post 57 is provided.
   The cam portion 56 is a portion made of a small piece disposed between the posterior condyle 22 of the medial condyle 6 and the posterior condyle 32 of the lateral condyle 7. The cam portion 56 is in contact with the post 57 of the tibial plate 4A when the flexion angle θ of the knee is a predetermined angle or more, thereby guiding flexion of the knee. The cam portion 56, the medial condyle 6, and the lateral condyle 7 define an opening portion 58. The post 57 is inserted into the opening portion 58. The post 57 is a portion in the shape of a column disposed between the medial recess 53 and the lateral recess 54 of the plate 51.
(2) Although the foregoing embodiment was described using an example in which the third joint face and the fourth joint face of the tibial plate are bilaterally symmetrical to each other, there is no limitation to this.
(3) The amount of decrease in the distance between the second contact portion on the second medial pivot guide portion and the reference face per unit flexion angle, in accordance with an increase in the flexion angle, may vary according to the position on the second medial pivot guide portion along the displacement direction. In a similar manner, the amount of decrease in the distance between the first contact portion on the first medial pivot guide portion and the reference face per unit flexion angle, in accordance with an increase in the flexion angle, may vary according to the position on the first medial pivot guide portion along the displacement direction. Note that the first medial pivot guide portion of the first joint face may be configured such that a change in the distance between the first contact portion and the reference face in accordance with a change by unit flexion angle is zero.
(4) Although the foregoing embodiment was described using an example in which the first medial pivot guide portion and the second medial pivot guide portion are in contact with the tibial plate when the flexion angle is at least a predetermined reference value that is greater than zero, there is no limitation to this. For example, the first medial pivot guide portion and the second medial pivot guide portion may be always in contact with the tibial plate.
(5) Although the foregoing embodiment was described using an example in which the top portion of the first joint face and the top portion of the second joint face have a plurality of radii of curvature when viewed from a side, there is no limitation to this. For example, the first joint face and the second joint face may have a single radius of curvature when viewed from a side.

### Industrial Applicability

The present invention is widely applicable as an artificial knee joint implant for use in surgery for replacing a knee joint of a patient by an artificial knee joint.

### List of Reference Numerals

- 1: Artificial knee joint implant
- 2: Femoral component
- 4: Tibial plate
- 11: First joint face
- 12: Second joint face
- 41: First contact portion
- 42: Second contact portion
- 43: Reference face
- 46: First medial pivot guide portion
- 48: Second medial pivot guide portion
- 101: Femur
- 102: Tibia
- 103: Distal portion
- 104: Proximal portion
- 105: Medial collateral ligament
- 106: Lateral collateral ligament
- D1, D2: Distance
- ΔD1, ΔD2: Amount of decrease in distance
- L1: Axial line of tibia

## Claims

1. An artificial knee joint implant for use in surgery for replacing a knee joint of a patient by an artificial knee joint, comprising:
a femoral component that is to be attached to a distal portion of a femur of the patient; and
a tibial plate that is to be attached to a proximal portion of a tibia of the patient, and guides flexion of the femur relative to the tibia in cooperation with the femoral component;
wherein the femoral component includes a first joint face in the shape of a projecting curve that is in contact with the tibial plate at a position adjacent to a medial collateral ligament of the patient, and a second joint face in the shape of a projecting curve that is in contact with the tibial plate at a position adjacent to a lateral collateral ligament of the patient, the first joint face and the second joint face being arranged side by side in a left-right direction,
a predetermined reference face extending orthogonal to the left-right direction is defined between the first joint face and the second joint face,
the first joint face and the second joint face respectively include a first medial pivot guide portion and a second medial pivot guide portion for guiding a medial pivot motion of the femoral component about an axial line of the tibia,
a first contact portion and a second contact portion, which are portions in contact with the tibial plate, of the first joint face and the second joint face are displaced on the first joint face and the second joint face in accordance with a change in a flexion angle of the femur relative to the tibia, and
an amount of decrease in a distance between the second contact portion on the second medial pivot guide portion and the reference face in accordance with an increase per unit flexion angle, which is a unit amount of the flexion angle, is greater than an amount of decrease in a distance between the first contact portion on the first medial pivot guide portion and the reference face in accordance with the increase per unit flexion angle.

2. The artificial knee joint implant according to claim 1,
wherein the tibial plate includes a third joint face that is in contact with the first joint face, and a fourth joint face that is in contact with the second joint face, and
the third joint face and the fourth joint face are bilaterally symmetrical to each other.

3. The artificial knee joint implant according to claim 1 or 2, wherein the amount of decrease in the distance between the second contact portion on the second medial pivot guide portion and the reference face per unit flexion angle, in accordance with an increase in the flexion angle, is the same throughout the second medial pivot guide portion.

4. The artificial knee joint implant according to any one of claims 1 to 3, wherein the amount of decrease in the distance between the first contact portion on the first medial pivot guide portion and the reference face per unit flexion angle, in accordance with an increase in the flexion angle, is the same throughout the first medial pivot guide portion.

5. The artificial knee joint implant according to any one of claims 1 to 4, wherein the first medial pivot guide portion and the second medial pivot guide portion come into contact with the tibial plate when the flexion angle is at least a predetermined reference value that is greater than zero.

6. The artificial knee joint implant according to claim 5, wherein, when the flexion angle is less than the reference value, the distance between the first contact portion and the reference face is constant, and the distance between the second contact portion and the reference face is constant, regardless of the flexion angle.
